(19) Europäisches Patentamt
European Patent Office
Office européen des brevets

(11) Publication number: **0 037 588**
**B1**

(12) **EUROPEAN PATENT SPECIFICATION**

(45) Date of publication of patent specification: **05.09.84**

(51) Int. Cl.$^3$: **C 07 D 307/42**

(21) Application number: **81102712.7**

(22) Date of filing: **09.04.81**

(54) **Method of preparing furfuryl alcohols.**

(30) Priority: **09.04.80 JP 47329/80**

(43) Date of publication of application:
**14.10.81 Bulletin 81/41**

(45) Publication of the grant of the patent:
**05.09.84 Bulletin 84/36**

(84) Designated Contracting States:
**BE CH DE FR GB IT LI NL SE**

(56) References cited:
**DE-A-2 827 383**

**Tetrahedron 34, 2775-2778 (1978)**

**Journal Org. Chem. 28, 3269-3272 (1963)**

**Houben-Weyl, Methoden der Organischen Chemie Vol. XIII/2a,p. 65(1973)**

**The file contains technical information submitted after the application was filed and not included in this specification**

(73) Proprietor: **SUMITOMO CHEMICAL COMPANY, LIMITED**
**15 Kitahama 5-chome Higashi-ku**
**Osaka-shi Osaka-fu (JP)**

(72) Inventor: **Takisawa, Yukihisa**
**1-2-40, Hirata Ibaraki**
**Osaka (JP)**
Inventor: **Saito, Kenji**
**2-10-3-321, Sonehigashi-machi Toyonaka**
**Osaka (JP)**
Inventor: **Yamachika, Hiroshi**
**2-1, Kuwata-cho Ibaraki**
**Osaka (JP)**

(74) Representative: **Vossius Vossius Tauchner Heunemann Rauh**
**Siebertstrasse 4 P.O. Box 86 07 67**
**D-8000 München 86 (DE)**

(56) References cited:

**Fieser and Fieser, Reagents for Organic Synthesis, p. 415, 418 and 1140 (1967)**

**Practical Organic Chemistry, A.I. Vogel, p. 521 and 522 (1964)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

## Description

The present invention relates to an improved process for the production of furfuryl alcohols. More particularly, it relates to a method for producing furfuryl alcohols of the formula:

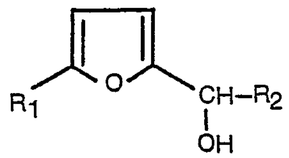

(I)

wherein $R_1$ is a hydrogen atom or a methyl group and $R_2$ is an allyl or $\alpha$-methylallyl group.

Furfuryl alcohols of the formula (I) are useful intermediates for the synthesis of agricultural chemicals, medicines, perfumes and the like.

In producing alcohols from carbonyl compounds by the so-called Grignard reaction it is common practice to first prepare the Grignard reagent and then allow it to react with the carbonyl compound. For instance, G. Piancatelli et al., "Tetrahedron", Vol. 34 (1978), 2775—2778 disclose a method for producing 5-methyl-$\alpha$-allylfurfuryl alcohol wherein the objective alcohol is obtained by first reacting allyl bromide with magnesium to obtain allylmagnesium bromide, which is then allowed to react with 5-methylfurfural, followed by hydrolysis.

Allyl halides, however, have a higher activity than other common halides and easily cause side reactions like the Wurtz reaction. Therefore, it is extremely difficult to obtain their Grignard reagents in high yields. In addition, when low-cost allyl chloride is used as the allyl halide, vigorous stirring is necessary for the progress of the reaction because the resulting allylmagnesium chloride is insoluble in diethyl ether cf. J. Org. Chem., Vol. *9* (1944), 359—372. It is therefore also difficult to adapt this method to the production on an industrial scale.

An alternative method was also studied, as shown in J. Org. Chem., Vol. *28* (1963), 3269—3272 wherein a carbonyl compound and allyl chloride are simultaneously added to a reaction system comprising diethyl ether as a solvent. The application of this method to allyl chloride and 5-methyl-furfural has the disadvantage that the Grignard reaction product precipitates due to its insolubility in diethyl ether, which causes an increase in the viscosity of the reaction system. Therefore, this method is industrially disadvantageous in terms of stirring efficiency, removal of reaction heat and working up of the reaction mixture.

The preparation of $\alpha$-methallyl-2-furanmethanol in 81% yield by the Barbier-Grignard procedure, i.e., the simultaneous addition of a solution of methallyl chloride and a solution of furfural in diethyl ether to a suspension of magnesium turnings in diethyl ether is known from DE—A—2 827 383.

The use of tetrahydrofuran and similar cyclic ethers as solvents in Grignard-syntheses is mentioned in Houben-Weyl, Methoden der Organischen Chemie, Vol. XIII/2a, page 65 (1973). Furthermore it is stated there that unsaturated and polar halides may be converted in better yields into Grignard compounds when using furan or 5,6-dihydro-4H-pyran instead of diethyl ether. These solvents are more expensive than diethyl ether. Only one to three equivalents per mole halide are necessary if benzene, toluene, xylene or naphthalene are used as diluents.

From Fieser and Fieser, Reagents for Organic Synthesis, page 415, (1967), a solution of methyl magnesium bromide in tetrahydrofuran-benzene is known as a commercial reagent. On page 418 it is stated that vinyl chloride and vinyl bromide readily form vinyl magnesium halides in solution in tetrahydrofuran. Benzene and xylene are mentioned as co-solvents to an ethereal solution of Grignard reagent. Grignard reagents in high yield are obtained in ligroin, toluene or xylene by first adding 1 mole of tetrahydrofuran for each mole of magnesium; see also page 1140.

It has now been found that in the above reaction between allyl chloride and 5-methylfurfural the use of tetrahydrofuran or a mixture of tetrahydrofuran and an aromatic hydrocarbon as a solvent can prevent precipitation of the reaction product, which leads to high yields of the final product on an industrial scale. The present invention is based on this finding.

The invention, therefore, relates to a process for preparing furfuryl alcohols of the formula I:

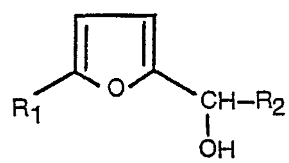

(I)

wherein $R_1$ is a hydrogen atom or a methyl group and $R_2$ is an allyl or $\alpha$-methylallyl group, by reacting the corresponding furfural of the formula II:

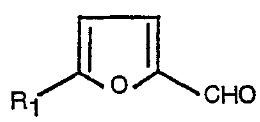

(II)

wherein $R_1$ is as defined above, with magnesium and allylchloride or $\alpha$-methylallyl chloride and hydrolysing the resulting product, characterized in that tetrahydrofuran or its mixture with benzene, toluene, xylene or mixtures thereof is used as a reaction medium, and the furfural and allyl chloride or $\alpha$-methylallyl chloride are simultaneously added to the reaction medium containing magnesium.

The use of tetrahydrofuran or of a mixture of tetrahydrofuran and benzene, toluene or xylene or mixtures thereof is essential to accomplish the object of the present invention. Tetrahydrofuran is considered to be the most suitable solvent for the reaction of the invention since both organic reactants and the Grignard reaction products are readily soluble therein.

When tetrahydrofuran is used as the only solvent, its amount may be kept constant or varied during the reaction and is preferably not less than 2.0 parts by weight based on one part by weight of the starting furfural of the formula (II). A larger amount of tetrahydrofuran can be used without limitation provided it does not adversely affect the reaction. Since the Grignard reaction product is readily soluble in tetrahydrofuran, it does not separate out from the reaction medium even at –30°C. This is industrially quite advantageous in that large temperature differences between a cooling medium and the reaction system can be accommodated, especially vigorous stirring is not required and the reaction mixture can be transported without difficulty.

On the one hand, however, it is desirable to reduce the amount of tetrahydrofuran as much as possible because its recovery from the reaction mixture in a pure form is not easy, as explained below. On the other hand, an excessive reduction of the amount of tetrahydrofuran is less desirable because the viscosity of the reaction mixture increases and the organic reactants and Grignard reaction product may become insoluble in the reaction mixture.

Accordingly, the combined use of tetrahydrofuran with benzene, toluene or xylene or mixtures thereof (aromatic hydrocarbon) is greatly preferred.

When tetrahydrofuran is used in combination with the aromatic hydrocarbon, the amount of tetrahydrofuran is preferably not less than 0.5 parts by weight based on one part by weight of the starting furfural of the formula (II). The amount of the aromatic hydrocarbon is usually more than about 1.0 part by weight based on one part by weight of the starting material, although it varies somewhat depending on the nature of the aromatic hydrocarbon used. An excessive amount of the aromatic hydrocarbon can be used without limitation provided the reaction is not adversely affected. By the combined use of tetrahydrofuran and the aromatic hydrocarbon it becomes possible, as aforementioned, to reduce the amount of tetrahydrofuran and yet to prevent precipitation of the Grignard reaction product in crystalline form, whereby the removal of heat of reaction, stirring of the reaction mixture and transportation of the reaction products can be carried out with ease.

Since tetrahydrofuran is soluble in water and forms an azeotropic mixture therewith, dehydration and re-use of tetrahydrofuran recovered from the reaction mixture requires extraction distillation and treatment with a suitable dehydrating agent. However, the combined solvent mentioned above does not cause any problems in the dehydration and the re-use of the solvent.

Also, it is very important for the accomplishment of the object of the present invention to add to the reaction medium containing magnesium a furfural of the formula (II) and allyl chloride or $\alpha$-methylallyl chloride simultaneously. Both compounds may be added to the reaction medium individually or as a mixture, usually in the form of a solution in tetrahydrofuran or a mixture of tetrahydrofuran and the aromatic hydrocarbon. The simultaneous addition of the organic reactants is particularly advantageous for industrial scale production in the following respects: the Grignard reaction products can be obtained in high yield even at elevated reaction temperatures; the reaction mixture can be efficiently cooled because it contains no precipitated crystals; the reaction time can be markedly shortened; and even a small excessive amount of magnesium and allyl chloride or $\alpha$-methylallyl chloride are sufficient to complete the reaction.

The term "simultaneously" herein used is intended to mean not that the reactants are added instantaneously, but that the furfural of the formula (II) and allyl chloride or $\alpha$-methylallyl chloride are added continuously or intermittently without extreme previous addition of either one of them. Thus, addition time and addition rate are not critical.

In the present invention the reaction temperature may be within a range of –20°C to the refluxing temperature (66°C) of tetrahydrofuran when used alone or within a range of –20 to 60°C in the combined use of tetrahydrofuran and the aromatic hydrocarbon. In either case a range of 0 to 40°C is more preferred. A too low reaction temperature, however, increases the viscosity of the reaction mixture and requires a very efficient cooling. The reaction pressure is not particularly limited. However, it is desirable to carry out the reaction under redcued pressure whereby the removal of the reaction heat by refluxing and control of the reaction temperature becomes possible.

The amount of each starting compound may be determined based on the amount of one starting material which is desired to be completely consumed during the reaction. However, a small excess is sufficient for the others. This is very advantageous in reducing costs for starting material as compared with the conventional method in which, for obtaining allyl-magnesium chloride or $\alpha$-methylallylmagnesium chloride, 1.3 to 2.2 moles of each of allyl chloride or $\alpha$-methylallyl chloride and magnesium is used based on 1 mole of the carbonyl compound, The material to be completely consumed may be selected

taking into account various conditions such as effects on subsequent steps and reaction equipment. For example, complete consumption of magnesium is industrially advantageous because it avoids the removal or recovery of unreacted magnesium from the reaction mixture.

In the Grignard reaction of the present invention it is desirable to start the reaction, before charging the furfural of the formula (II) and allyl chloride or $\alpha$-methylallyl chloride, by adding a small amount of allyl chloride or $\alpha$-methylallyl chloride and, if necessary, iodine to tetrahydrofuran or a mixture of tetrahydrofuran and the aromatic hydrocarbon containing magnesium. After completion of the Grignard reaction, the resulting product is hydrolyzed to obtain the objective furfuryl alcohol of the formula (I).

The condition of hydrolysis is not particularly limited, and the hydrolysis can easily be carried out by conventional methods using an aqueous solution of ammonium chloride, hydrochloric acid, sulfuric acid or a mixture thereof. The hydrolyzed product thus obtained may be purified, e.g., by distillation.

In this way, according to the present invention, the furfuryl alcohols of the formula (I) is obtained in high yield and in an industrially very advantageous way.

The present invention will be illustrated in more detail with reference to the following examples, wherein % is by weight unless otherwise indicated.

Example 1

Into a dried 300-ml volume round-bottom flask, there were charged magnesium turnings (8.46 g), dry tetrahydrofuran (132.0 g) and iodine (20 mg), and allyl chloride (1.5 g) was dropwise added thereto while stirring at room temperature. The reaction mixture was allowed to stand for 30 minutes. The beginning of the reaction was confrimed by the disappearance of iodine color and by generation of heat.

Then, a solution comprising 5-methylfurfural (33.0 g), allyl chloride (27.9 g) and tetrahydrofuran (66.0 g) was dropwise added at 66°C (refluxing temperature) in 1 hour while stirring, and stirring was continued at the same temperature for 30 minutes. After completion of the reaction, the resultant mixture and a 14.1% aqueous sulfuric acid (116.4 g) were simultaneously poured into water (100 g) at 10°C in 30 minutes while stirring. Stirring was continued at the same temperature for 1 hour.

After completion of the reaction, the resulting solution was separated into an aqueous and an oily layer. The aqueous layer was discarded. From the oily layer the solvent was removed by evaporation, and the residue was vacuum-distilled (85°C/6 mmHg) to give 41.1 g of 2-(1-hydroxy-3-butenyl)-5-methylfuran. The yield was 90.0% based on 5-methylfurfural and 78.1% based on magnesium.

Example 2

The initial reaction was carried out in the same manner as in Example 1. Under stirring, a solution comprising 5-methylfurfural (33.0 g) and allyl chloride (27.9 g) was then dropwise added to the reaction system at 0°C in 0.5 hour while cooling with an acetone/dry ice bath of −60°C. The reaction mixture was then allowed to stand at the same temperature for 1.5 hours while stirring at 10°C for 30 minutes. Stirring the resultant solution was dropwise added to a 13.8% aqueous ammonium chloride (232.1 g) while stirring at 10°C for 30 minites. Stirring was continued at the same temperature for 1 hour.

After completion of the reaction, the resulting solution was treated in the same manner as in Example 1 to give 43.9 g of 2-(1-hydroxy-3-butenyl)-5-methylfuran. The yield was 96.1% based on 5-methylfurfural and 83.4% based on magnesium.

Example 3

The initial reaction was carried out in the same manner as in Example 1 except that the amount of magnesium turnings was changed to 8.02 g.

Under stirring, a solution comprising 5-methylfurfural (33.0 g) and allyl chloride (27.9 g) was dropwise added to the reaction system at 10°C in 15 hours. The reaction mixture was then allowed to stand at the same temperature for 1.5 hours while stirring. After completion of the reaction, the resultant solution was dropwise added to a solution of ammonium chloride (32.1 g) in a 5.17% aqueous hydrochloric acid (210.9 g) while stirring at 10°C for 30 minutes. Stirring was continued at the same temperature for 1 hour.

After completion of the reaction, the resulting solution was treated in the same manner as in Example 1 to give 42.7 g of 2-(1-hydroxy-3-butenyl)-5-methylfuran. The yield was 93.5% based on 5-methylfurfural and 85.0% based on magnesium.

Example 4

Into a reactor were charged, under a nitrogen stream, magnesium turnings (13.5 g), tetrahydrofuran (27.5 g; water content, 300 ppm) and toluene (27.5 g). Allyl chloride (2.8 g) was dropwise added thereto while stirring at room temperature. The reaction mixture was allowed to stand for 5 minutes. The beginning of the reaction was confirmed by heat generation.

Then, a solution comprising 5-methylfurfural (55.0 g), allyl chloride (43.1 g) and toluene (165.2 g) was dropwise added at 40°C within 4 hours while stirring, and the reaction mixture was then kept at the same temperature for 1 hour. After completion of the reaction, the resulting mixture and a 15.0% aqueous sulfuric acid (147.1 g) were simultaneously poured into water (211 g) at 40°C in 30 minutes under

stirring, while maintaining a pH of the reaction medium at above 8. Thereafter, a 20% aqueous acetic acid (30 g) was dropwise added therein to lower the pH to 5, and the mixture was kept at the same temperature for 1 hour.

After completion of the reaction, the resulting solution was separated into an aqueous and an oily layer. The aqueous layer was extracted with toluene (150 g). The toluene layer was combined with the oily layer and washed with a 5% aqueous sodium carbonate solution. The subsequent solvent removal by evaporation from the oily layer and vacuum-distillation (95—97°C/1.33 x $10^3$ Pa.) of the residue gave 74.6 g of 2-(1-hydroxy-3-butenyl)-5-methylfuran. The yield was 98,0% based on 5-methylfurfural and 88.2% based on magnesium.

Example 5

Into a reactor were charged, under a nitrogen stream, magnesium turnings (14.58 g), tetrahydrofuran (48.0 g; water content, 300 ppm) and iodine (0.15 g). $\alpha$-Methylallyl chloride (3.26 g) was dropwise added thereto while stirring at room temperature. The reaction mixture was allowed to stand for 5 minutes. The beginning of the reaction was confirmed by the disappearance of iodine color and by generation of heat.

Then, a solution comprising furfural (48.0 g), $\alpha$-methylallyl chloride (51.7 g) and *xylene* (168.0 g) was dropwise added at 15°C within 3 hours while stirring, and the raction mixture was then kept at the same temperature for 2 hours. After completion of the reaction, the resultant mixture and a 15.0% aqueous sulfuric acid (147.1 g) were simultaneously poured into water (211 g) at 20°C in 12 minutes under stirring, while maintaining a pH of the reaction medium at above 8. Thereafter, a 20% aqueous acetic acid (30 g) was dropwise added thereto to lower the pH to 5, and the system was kept at the same temperature for 45 minutes.

After completion of the reaction, the resulting solution was separated into an aqueous and an oily layer. The aqueous layer was extracted with xylene (150 g). The xylene layer was combined with the oily layer and washed with a 5% aqueous sodium carbonate solution. The subsequent solvent removal by evaporation from the oily layer and vacuum-distillation (90—93.5°C/2.0x$10^3$ Pa.) from the residue gave 75.1 g of 2-(1-hydroxy-2-methyl-3-butenyl)furan. The yield was 98.7% based on furfural and 82.2% based on magnesium.

Comparative Example 1

Into a reactor were charged, under a nitrogen stream, magnesium turnings (13.5 g), tetrahydrofuran (55.1 g; water content, 300 ppm) and iodine (0.15 g). Allyl chloride (2.8 g) was dropwise added thereto while stirring at room temperature. The reaction mixture was allowed to stand for 5 minutes. The beginning of reaction was confirmed by the disappearance of iodine color and by generation of heat.

Then, a solution comprising allyl chloride (43.1 g) and toluene (165.2 g) was dropwise added to the mixture at 40°C in 4 hours while stirring. 5-Methylfurfural (55.0 g) was then dropwise added at the same temperature for 1 hour under stirring. The reaction mixture was kept at the same temperature for 1 hour. After completion of the reaction, the resultant mixture and a 15.0% aqueous sulfuric acid (121 g) were simultaneously poured into water (211 g) at 40°C in 30 mintues under stirring, while maintaining a pH of the reaction medium at above 8. Thereafter, a 20% aqueous acetic acid (5 g) was dropwise added thereto to lower the pH to 5, and the mixture was kept at the same temperature for 1 hour.

After completion of the reaction, the resulting solution was treated in the same manner as in Example 4 to give 46.6 g of 2-(1-hydroxy-3-butenyl)-5-methylfuran. The yield was 61.2% based on 5-methylfurfural and 55.1% based on magnesium. The conversion of the starting 5-methylfurfural was 90%.

**Claims**

1. Process for preparing furfuryl alcohols of the formula I:

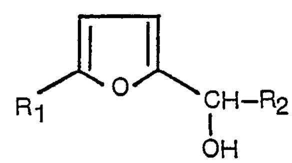

(I)

wherein $R_1$ is a hydrogen atom or a methyl group and $R_2$ is an allyl or $\alpha$-methylallyl group, by reacting the corresponding furfural of the formula II:

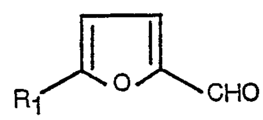

(II)

wherein $R_1$ is as defined above, with magnesium and allylchloride or $\alpha$-methylallyl chloride and hydrolysing the resulting product, characterized in that tetrahydrofuran or its mixture with benzene, toluene or xylene or mixtures thereof is used as a reaction medium, and the furfural and allyl chloride or $\alpha$-methylallyl chloride are simultaneously added to the reaction medium containing magnesium.

2. Process in accordance with claim 1, characterized in that tetrahydrofuran when used as the only solvent is used in an amount of not less than 2.0 parts by weight based on one part by weight of the starting furfural.

3. Process in accordance with claim 1, characterized in that tetrahydrofuran when used in combination with benzene, toluene or xylene or mixtures thereof is used in an amount of not less than 0.5 parts by weight based on one part

by weight of the starting furfural.

**Patentansprüche**

1. Verfahren zur Herstellung von Furfurylalkoholen der Formel I

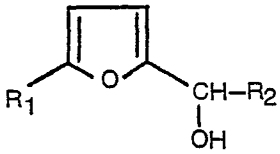

(I)

in der $R_1$ ein Wasserstoffatom oder eine Methylgruppe und $R_2$ eine Allyl- oder $\alpha$-Methylallylgruppe bedeuten, durch Umsetzen des entsprechenden Furfurals der Formel II

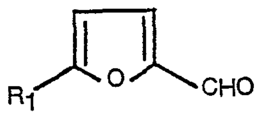

(II)

in der $R_1$ die vorstehend angegebene Bedeutung hat, mit Magnesium und Allylchlorid oder $\alpha$-Methylallylchlorid und Hydrolysieren des erhaltenen Produkts, dadurch gekennzeichnet, daß man Tetrahydrofuran oder sein Gemisch mit Benzol, Toluol oder Xylol oder deren Gemische als Reaktionsmedium einsetzt und das Furfural und das Allylchlorid oder $\alpha$-Methylallylchlorid gleichzeitig im Reaktionsmedium zusetzt, das Magnesium enthält.

2. Verfahren nach Anspruch 1, dadurch gekennzeichnet, daß man Tetrahydrofuran alleine als Lösungsmittel in einer Menge von nicht weniger als 2,0 Gewichtsteile bezogen auf 1 Gewichtsteil des Ausgangsproduktes Furfural verwendet.

3. Verfahren nach Anspruch 1, dadurch gekennzeichnet, daß bei Verwendung von Tetrahydrofuran in Kombination mit Benzol, Toluol oder Xylol oder deren Gemische dieses in einer Menge von nicht weniger als 0,5 Gewichtsteile, bezogen auf 1 Gewichtsteil des Ausgangsproduktes Furfural einsetzt.

**Revendications**

1. Procédé pour préparer des alcools furfuryliques ayant la formule:

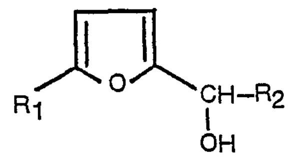

(I)

où $R_1$ est un atome d'hydrogène ou un groupe méthyle et $R_2$ est un groupe allyle ou $\alpha$-méthylallyle, en faisant réagir le furfural correspondant de formule II:

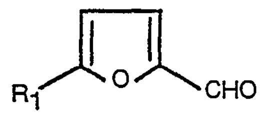

(II)

où $R_1$ est comme défini ci-dessus, avec du magnésium et du chlorure d'allyle ou du chlorure d'$\alpha$-méthylallyle et en hydrolysant le produit résultant, caractérisé en ce que le tétrahydrofurane ou son mélange avec le benzène, le toluène ou le xylène ou leurs mélanges est employé comme milieu réactionnel, et le furfural et le chlorure d'allyle ou le chlorure d'$\alpha$-méthylallyle sont simultanément ajoutés au milieu réactionnel contenant du magnésium.

2. Procédé selon la revendication 1, caractérisé en ce que le tétrahydrofurane, quand on l'utilise comme seul solvant, est employé en quantité non inférieure à 2,0 parties en poids en se basant sur une partie en poids de furfural de départ.

3. Procédé selon la revendication 1, caractérisé en ce que le tétrahydrofurane, quand on l'utilise en combinaison avec du benzène, du toluène ou du xylène ou leurs mélanges est utilisé en quantité non inférieure à 0,5 partie en poids en se basant sur une partie en poids de furfural de départ.